Europäisches Patentamt

European Patent Office

Office européen des brevets

(11) Publication number: 0 352 894 A2

# EUROPEAN PATENT APPLICATION

(21) Application number: 89306057.4

(22) Date of filing: 15.06.89

(51) Int. Cl.⁴: C12N 5/00 , C12N 15/00 , C12P 21/00 , A61K 7/00

(30) Priority: 17.06.88 GB 8814476

(43) Date of publication of application:
31.01.90 Bulletin 90/05

(84) Designated Contracting States:
AT BE CH DE ES FR GB GR IT LI NL SE

(71) Applicant: UNILEVER PLC
Unilever House Blackfriars
London EC4P 4BQ(GB)
(84) GB

Applicant: UNILEVER NV
Burgemeester s'Jacobplein 1 P.O. Box 760
NL-3000 DK Rotterdam(NL)
(84) BE CH DE ES FR GR IT LI NL SE AT

(72) Inventor: Bayley, Susan Ann
10 Furness Close
Bedford, MK41 8RN(GB)

(74) Representative: Tonge, Robert James et al
UNILEVER PLC Patents Division P.O. Box 68
Unilever House
London EC4P 4BQ(GB)

(54) Production of cosmetic or pharmaceutical composition.

(57) Dermal papillae cells are immortalized and cultured to produce hair growth promoter. The hair growth promoter is then collected and used to prepare compositions for application to the human skin.

EP 0 352 894 A2

## PRODUCTION OF COSMETIC OR PHARMACEUTICAL COMPOSITION

This invention relates to a cosmetic or pharmaceutical composition for topical application to mammalian skin, and to the means for producing a constituent of such composition. This constituent is a hair growth promoter (which may be a mixture of materials) capable of promoting hair growth.

Our pending European application 87.311316.1, due to be published in June 1988, discloses a hair growth promoter intended to be incorporated in a composition applied topically to increase human hair growth. This hair growth promoter can be obtained from a cell-free supernatant of cultured dermal papillae fibroblasts; it is able to promote hair growth in the rat, is proteinaceous, and is further characterised by:

a) having an apparent molecular weight of at least 500 Daltons; and

b) possessing the ability to initiate DNA synthesis in a culture of serum starved NIH 3T3 cells.

We have now found that dermal papillae cells can be transformed (transfected) by recombinant DNA techniques to render them immortal, allowing unlimited growth potential in culture, while retaining the desired cell phenotype. The consequence is that it is practical to allow the cells to reproduce more times than would otherwise be possible - perhaps more than 10 times - while retaining desired phenotype. Such cells can be utilised to produce hair growth promoter, which may possibly be a mixture of materials and desirably is or includes the material according to our European application mentioned above.

In a first aspect therefore, this invention provides dermal papillae cells transformed (transfected) by incorporation of an immortalizing gene into the genetic information of the cells.

The immortalizing gene may come from an oncogenic virus, such as polyoma virus, although other sources of immortalizing gene are not excluded.

In a second aspect the invention provides a method of making immortalized dermal papillae cells which comprises transforming (transfecting) the cells by recombinant DNA technology to incorporate an immortalizing gene into the genetic information of the cells.

In a third aspect this invention provides the production of hair growth promoter by means of immortalized dermal papillae cells.

This production will generally amount to a method of making hair growth promoter by culturing dermal papillae cells according to the first aspect of this invention, or obtained by the method of the second aspect, under conditions for the cells to produce hair growth promoter.

The invention also includes culture supernatant, and compositions, which contain the hair growth promoter.

A particular embodiment of the present invention will now be described, with reference to the following drawings, in which

Figure 1 is restriction maps of the plasmids pZIP-Neo SV(X)1 and pPyLT1, showing the cleavage sites of the restriction endonucleases mentioned in the text and figures;

Figure 2 is a schematic diagram showing the construction of the retroviral vector, pZIT1, as described in the text; and

Figure 3 is a map of selected restriction sites of a pZIT1 construct, in which the large T gene is in the "sense" direction.

A preferred method of producing immortalized dermal papillae cells according to the present invention is described hereinafter.

This method describes the immortalization of specialised primary cells, namely dermal papillae cells, with an immortalizing gene, which is the polyoma virus large T gene. High frequencies of gene transfer are achieved using recombinant defective retroviruses.

To summarise the procedure: a known vector was employed, which contained retroviral control sequences, a viral packaging signal, a unique cloning site for foreign gene insertion (BamHI) and a selectable dominant marker (resistance to the antibiotic G418). This vector was modified to incorporate the large T gene and the modified vector was then used in conjunction with a cell line (the Ψ2 cell line) which contains a packaging mutant Moloney murine leukaemia virus in its proviral state. This virus contained all of the retroviral structural genes but no packaging information. The modified vector was cloned and introduced into the Ψ2 cell line by calcium phosphate coprecipitation and as a result of its packaging signal was able to make use of the viral structural proteins to produce defective retroviruses. Virus particles were harvested and used to infect dermal papillae cells, resulting in high frequencies of gene transfer.

A more detailed description now follows.

## a) Construction of pZIT Vectors

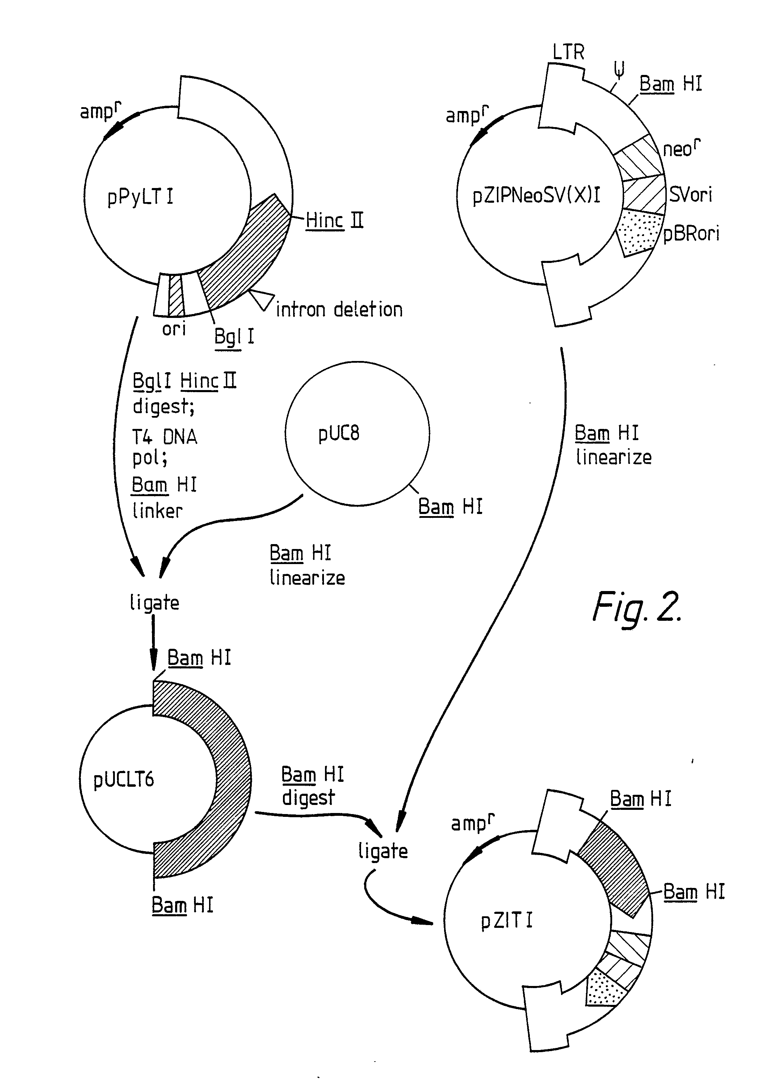

The large T gene of murine polyoma virus has been reported to exhibit immortalizing properties: Rassoulzadegan et al Proc. Natl. Acad. Sci. USA 80 4354-8 (1983). We inserted the large T gene into the known pZIP-Neo SV(X)1 plasmid in the manner shown in Figure 2, to obtain retroviral vectors. The pZIP-Neo SV(X)1 plasmid is described in Cepko et al, Cell 37 1053-62 (1984).

All plasmids were maintained and reproduced in E. Coli HB101 which is described in Boyer et al. J. Mol. Biol. 41 459-472 (1969).

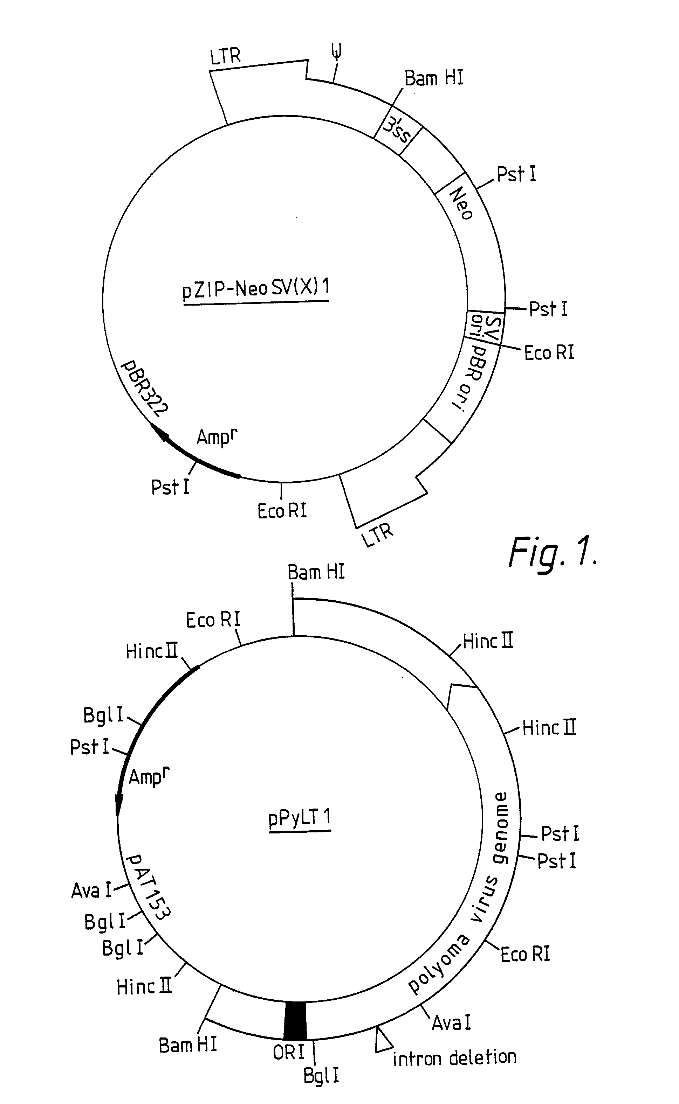

The process began with a recombinant plasmid pPyLT1 which is described by Zhu et al J. Virol. 51, 170-180 (1984). It is a pAT 153 vector containing the polyoma genome with an intron deletion, permitting transcription of the large T gene only (Figure 1). A 2491 base pair fragment, generated by restricting pPyLT1 with BglI and HincII was isolated and purified by standard methods as described in, for example, Maniatis et al, "Molecular Cloning, A Laboratory Manual", Cold Spring Harbor Laboratory (1982).

The 2491 base pair fragment was treated with T4 DNA polymerase to provide blunt ends, ligated to linkers and cloned into BamHI - linearised pUC8. Transformants were screened for insert-containing clones by plasmid isolation and restriction analysis. Three were found, and one designated pUCLT6 was used further.

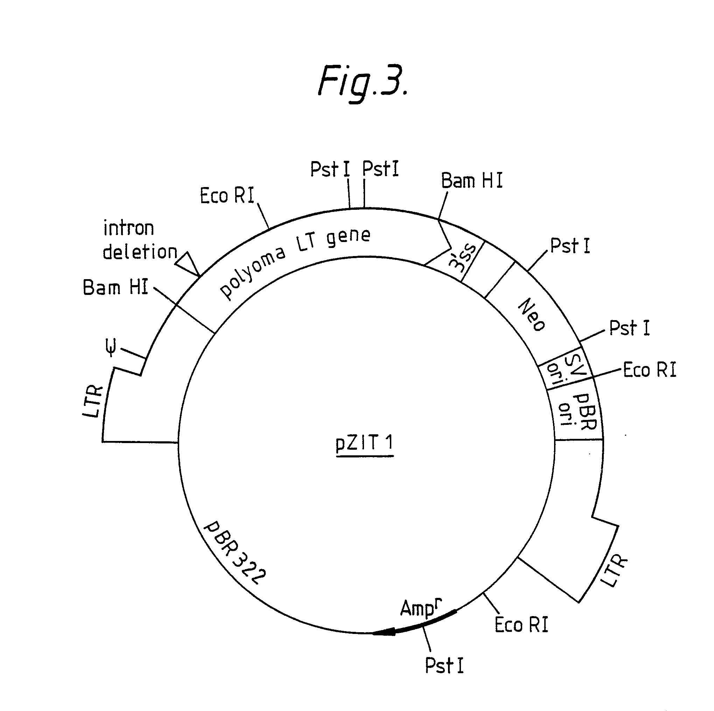

The recombinant plasmid pUCLT6 was cleaved with BamHI to generate both the large T gene and the pUC vector, and this mixture cloned into pZIP-Neo SV(X)1, with transformants being selected for kanamycin resistance to prevent pUC8 recombinants occurring. Insert-containing clones were initially screened by Southern blotting of transformant DNA (colony blotting), using as probe a [32]P nick-translated BglI - EcoRI fragment of large T. This was followed by plasmid isolation and restriction analysis of "positive" colonies. This revealed a number of recombinant plasmids including the construct which was used, pZIT1, an immortalizing vector with one copy of the large T gene in the sense orientation, and also a construct pαZIT which has one copy of the large T gene in the antisense orientation. Figure 3 details the restriction map of the pZIT1 construct.

b) Production of Ψ2:ZIT Cell Lines

pZIT1 was introduced into the Ψ2 cell line using a calcium phosphate coprecipitation technique as described in Wigler et al. Proc. Natl. Acad. Sci. USA 76 1373-6 (1979). The 2 cell line is described in Mann et al Cell 33 153-9 (1983). This introduction of pZIT1 produced a cell line Ψ2:ZIT. To carry this out Ψ2 cells were maintained in Dulbecco's modification of Eagle's Medium supplemented with 5% Foetal Calf Serum. 24h prior to transfection $2 \times 10^5$ Ψ2 cells were seeded in 25cm² tissue culture flasks. 3h prior to transfection the cells were given 5ml fresh medium per flask. 10μg pZIT1 was coprecipitated with calcium phosphate in a volume of 1ml and 500μl was added to each flask. After 4h incubation the cells were washed with tris buffered saline before incubating for a further 24h with fresh non-selective medium. The cultures were then trypsinised and $1 \times 10^5$ cells were seeded in 75cm² tissue culture flasks with medium containing 750μg/ml G418. The pZIT1 plasmid contains sequences derived from transposon Tn5 which encodes G418 resistance in animal cells and, after 10 days in selective medium, flasks of cells that had been transfected with plasmid DNA contained more than 100 clones. The clones in each individual flask were pooled and expanded into a cell line. Ψ 2:ZIT was one such cell line. In contrast, there were no live cells remaining in control flasks which contained cells that had only been treated with calcium phosphate precipitate.

c) Collection and Determination of Titre of Recombinant Defective Retrovirus

Ψ 2 cells that carry a vector based on pZIP-Neo SV(X)1 put recombinant defective retrovirus directly into the growth medium. For retrovirus collection, the cells were grown in antibiotic-free medium and, 24h before they reached confluence, the medium was replaced with 25ml fresh medium/175cm² tissue culture flask. After 24h incubation at 37°C the growth medium, which contained viral particles, was collected and passed through a 0.45μm filter to remove all cellular material before storage at -70°C.

The titres of virus stocks were determined using the following method. $4 \times 10^5$ 3T6 cells (a mouse fibroblast line) were seeded in 100mm tissue culture dishes. 24h later tenfold and hundredfold dilutions of each virus stock were prepared in complete medium and polybrene added to a final concentration of 8μg/ml. The medium was removed from the cells and 2ml virus dilution was added to each dish with each dilution done in duplicate. The dishes were incubated for 2h 30min at 37°C in an atmosphere of 5% $CO_2$ in air; the dishes were gently rocked every 30 min. 10ml fresh medium was then added to each dish. After a further 48h incubation period, $2 \times 10^5$ cells from each dish were transferred into duplicate dishes with medium containing 750μg/ml G418. After 10 to 14 days, clones were visible in dishes that had been infected with medium containing virus particles, but no live cells were left in dishes that had only been treated with growth medium or medium from Ψ 2 cells. After fixation with 1% glutaraldehyde, extraction with 100% methanol and staining with Giemsa, the clones were counted and titres calculated. Ψ 2:ZIT cells were found to produce viral particles at a titre of about $1 \times 10^4$

colony forming units per ml.

Using the method described above, a substantial fraction of 3T6 cells can be infected and become resistant to the antibiotic G418.

### d) Dissection of Dermal Papillae from Hair Follicles and Culture of Dermal Papillae Cells

Dermal papillae were isolated by microdissection from rat hair follicles.

The isolated papillae were maintained in a medium containing 15% by volume Foetal Calf Serum (FCS), the cells and medium being kept at 37°C in an atmosphere of 5% $CO_2$/95% air, in order to maintain a suitable pH value of from 6.5 to 7.5.

The medium employed was Dulbeccos' modified Eagle's Medium (DMEM) supplemented with 15% by volume FCS, as well as penicillin, streptomycin and kanamycin to reduce the risk of bacterial contamination.

Following attachment of the dermal papillae to the culture vessel, cells migrating from dissected papillae were allowed to grow for several weeks in the above nutrient medium, with regular changes of medium, before being passaged into fresh medium which omits the penicillin, streptomycin and kanamycin, to produce subconfluent monolayers of cells for infection with recombinant retrovirus. Passaging was achieved by removal of the medium, addition of warmed trypsin/EDTA solution (37°C) for about 5 minutes, detachment of rounded cells and 'splitting' the harvested cells into new culture vessels containing fresh medium with serum. In the presence of serum, most cells reattach and spread on the surfaces of the culture vessels, allowing the establishment of sparse monolayers.

### e) Gene Transfer Using Recombinant Defective Retrovirus

Sparse cultures of dermal papillae cells prepared by the culture method detailed above were allowed to expand into subconfluent monolayers in 24-well tissue culture plates. The cells are subconfluent and exposed to virus about 48 hours after the sparce cultures are prepared. The medium was then removed and 1ml undiluted virus stock containing 8μg/ml polybrene was added to each well. The plates were incubated for 2h 30mins at 37°C in an atmosphere of 5% $CO_2$ in air and the plates were gently rocked every 30min. For each well, the virus stock containing polybrene was then replaced with a fresh 0.5ml sample of virus stock diluted with 0.5ml fresh medium and the infection period continued for an additional 24 hours. The medium containing virus was then removed and the cells were incubated in fresh medium until they approached confluence. The cells from each well were harvested, split into several fresh wells and incubated as described above until subconfluent monolayers had formed when they were transferred to medium containing 200μg/ml G418. After several weeks (with regular changes of medium) clones were visible in wells where the cells had been infected with medium containing virus particles, but no live cells were left in wells that had been treated with medium from Ψ 2 cells.

Individual clones were picked and the cells passaged as described in d) using culture vessels with increasing surface areas until monoclonal cell lines were established. Alternatively, cells from a number of clones were pooled and, using the same method, expanded into polyclonal cell lines.

Indirect immunofluorescence staining with a rat monoclonal anti-large T, αPyLT-1 confirmed that the large T protein was expressed by cells infected with virus particles produced by Ψ 2:ZIT cells.

### f) Maintenance of Immortalized Cell Lines

Dermal papillae cells immortalized by gene transfer as described above, were maintained in tissue culture vessels containing Dulbeccos' modified Eagle's Medium (DMEM) supplemented with 15% FCS, and 200μg/ml G418. The cells were then repeatedly "passaged" into fresh medium as described in paragraph d) above, thus allowing the continuous propagation of the immortalized cell line.

### g) Cryopreservation

The dermal papillae cell lines were frozen in medium containing serum and a preservative such as 10 to 15% dimethyl sulphoxide, stored in liquid nitrogen and recovered from storage as described by Freshney in Culture of Animal Cells: A Manual of Basic Technique, Alan R. Liss, Inc, New York 190-198 (1983).

### h) Collection of Culture Supernatant

Immortalized cells were seeded in normal medium as at f) above. When they reached confluent or nearly confluent cultures the medium was changed to serum-free and G418-free medium, which was changed to fresh serum-free and G418-free medium after 24 hours. After a period of about 3 days, the culture supernatant was decanted from the cells and centrifuged or filtered to remove floating cells and cell debris, and then concentrated and dialysed against isotonic saline using, for example, an Amicon ultrafiltration device having a 500D, 2000D or 5000D (D = Dalton) molecular weight cut off.

The concentrated, dialysed culture supernatant containing the hair growth promoter can then be used in preparing a composition for promotion of hair growth as explained in our pending European application 87.311316. It can be used as such or it can be dried, for example, by freeze drying before dispersing in the vehicle.

## Claims

1. Dermal papillae cells transfected by incorporation of an immortalizing gene into the genetic information of the cells.

2. Cells according to claim 1 wherein the immortalizing gene is from an oncogenic virus.

3. Cells according to claim 2 wherein the immortalizing gene is from a polyoma virus.

4. A method of making immortalized dermal papillae cells which comprises transfecting the cells by recombinant DNA technology to incorporate an immortalizing gene into the genetic information of the cells.

5. A method according to claim 4 which comprises collecting and culturing dermal papillae cells, transfecting the cultured cells with recombinant vectors carrying an immortalizing gene, and selecting cells which have been transfected with the immortalizing gene.

6. A method according to claim 5 which comprises the steps of:

a) dissecting dermal papillae explants out of hair follicles;

b) maintaining and/or manipulating the explants in tissue culture so that individual dermal papillae cells migrate out of the explants;

c) maintaining and/or manipulating the dermal papillae cells in tissue culture to give subconfluent monolayers;

d) transfecting the cultured dermal papillae cells with one or more recombinant vectors carrying an immortalizing gene and a gene aiding selection of the transformants;

e) maintaining and/or manipulating the dermal papillae cells in tissue culture;

f) selecting dermal papillae cells which have been transfected by the incorporation of an immortalizing gene.

7. A method according to claim 6 further comprising the steps of:

g) maintaining and/or manipulating the selected transfected immortalized dermal papillae cells in tissue culture;

h) cryopreserving batches of the immortalized dermal papillae cells.

8. A method according to claim 6 or claim 7 wherein step (c) includes passaging the cells.

9. A method according to any one of claims 4 to 8 wherein the immortalizing gene is provided by an oncogenic virus.

10. A method according to claim 9 wherein the immortalizing gene is provided by a polyoma virus.

11. A method according to any one of claims 4 to 10 wherein the recombinant vector is a retrovirus.

12. Production of hair growth promoter by means of immortalizing dermal papillae cells.

13. A method of making hair growth promoter comprising utilising dermal papillae cells according to any one of claims 1 to 3 or produced by a method according to any one of claims 4 to 11, and culturing the said cells under conditions for the cells to produce hair growth promoter.

14. A method according to claim 13 which comprises the steps of:

a) maintaining immortalized dermal papillae cells in tissue culture;

b) maintaining the tissue culture conditions appropriate for the production of hair growth promoter.

15. A method according to claim 13 or claim 14 further comprising recovering the hair growth promoter from the tissue culture supernatant.

16. Culture supernatant containing hair growth promoter produced according to the methods of any one of claims 13 to 15.

17. A composition containing hair growth promoter produced according to any one of claims 13 to 15, and which is sufficient to increase hair growth in the rat, when applied thereto, by at least 10% more than that obtainable using a control composition from which the hair growth promoter has been omitted.

18. A composition according to claim 17 wherein at least a part of the hair growth promoter is characterised by:

i) being substantially proteinaceous;

ii) having an apparent molecular weight of at least 500 Daltons; and

iii) possessing the ability to initiate DNA synthesis in a culture of serum-starved NIH 3T3 cells.

19. A composition according to claim 17 or claim 18 in which the hair growth promoter forms from $1 \times 10^{-6}\%$ to 99% by weight, preferably $1 \times 10^{-3}\%$ to 90%.

20. A composition according to any of claims 17 to 19 which comprises a cosmetically acceptable vehicle in addition to the culture supernatant.

21. A composition according to any of claims 17 to 20 which additionally comprises an activity enhancer chosen from other hair growth stimulants, protein stabilising agents and penetration enhancers.

22. A composition according to claim 21, in which the other hair growth stimulant is chosen from minoxidil, minoxidil glucuronides, minoxidil sulphates or mixtures thereof.

23. A composition according to claim 21 or claim 22 in which the protein stabilising agent is chosen from glycerol, ethylenediaminetetraacetic acid, cysteine, $\alpha_2$-macroglobulin, serum or mixtures thereof.

24. A method of inducing cosmetic hair growth which comprises administering a composition according to claim 20.

_Fig. 1._

Fig. 2.

# Fig.3.